# EUROPEAN PATENT SPECIFICATION

(11) **EP 4 019 422 B1**
(45) Date of publication and mention of the grant of the patent: **08.11.2023**
(21) Application number: 21215737.4
(22) Date of filing: 17.12.2021
(51) Int. Cl.: B65D 21/02, A61J 1/16, A61M 5/00, A61J 7/00

(54) **A SPACER STRUCTURE FOR SEPARATING TWO STACKED TUBS CONFIGURED FOR THE STORAGE AND/OR TRANSPORT OF A PLURALITY OF MEDICAL CONTAINERS, AND A PACKAGING INCLUDING SAID SPACER STRUCTURE**
ABSTANDSHALTERSTRUKTUR ZUM TRENNEN VON ZWEI GESTAPELTEN WANNEN, DIE ZUR LAGERUNG UND/ODER ZUM TRANSPORT VON MEHREREN MEDIZINISCHEN BEHÄLTERN KONFIGURIERT SIND, SOWIE EINE VERPACKUNG MIT DIESER ABSTANDSHALTERSTRUKTUR
STRUCTURE D'ENTRETOISE POUR SÉPARER DEUX BACS EMPILÉS CONÇUS POUR LE STOCKAGE ET/OU LE TRANSPORT D'UNE PLURALITÉ DE CONTENEURS MÉDICAUX ET EMBALLAGE COMPRENANT LADITE STRUCTURE D'ENTRETOISE

(30) Priority: 23.12.2020 EP 20306677
(43) Date of publication of application: 29.06.2022
(73) Proprietor: Becton Dickinson France, 38800 Le Pont-de-Claix (FR)
(72) Inventor: EYMERY, Anaïs, 38450 Saint-Georges-de-Commiers (FR); LE LOC'H, Clémentine, 38240 Meylan (FR)
(74) Representative: Germain Maureau

(56) References cited:
- WO-A1-2009/053434
- WO-A1-2012/143533
- DE-A1- 10 204 291
- DE-U1-202017 100 104
- US-A1- 2015 344 179

## Description

The present invention relates to spacer structure for separating two stacked tubs configured for the storage and/or transport of a plurality of medical containers, and to a packaging including said spacer structure.

DE 202017100104 U, which accords with the preamble of claim 1, discloses a device for securing during transport a number of stacked objects forming at least a first layer and a second layer.

Medical containers such as pre-fillable or prefilled syringes are usually supported by a plate-shaped tray. This plate-shaped tray, also called a "nest", is placed in a box-shaped housing protecting the medical containers during storage or transport.

This box-shaped housing, also called a "tub", usually defines an opening for insertion or removal of the nest. The insertion or removal movement of the nest relative to the tub typically takes place along a vertical axis A. A cover film, such as a Tyvek^{®} sheet, seals the opening in order to keep sterility inside the tub and to maintain the medical containers inside the tub. This cover film is usually permeable to a sterilization gas such as ethylene oxide.

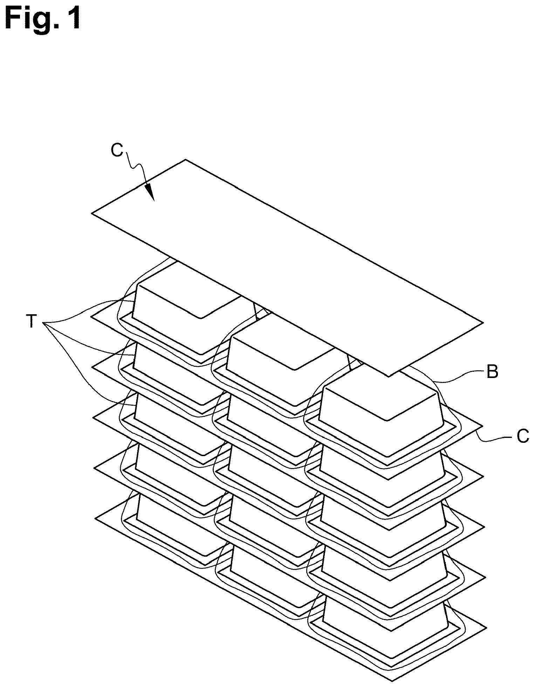

The tubs are usually disposed side by side and, as shown in Figure 1, a horizontal row of tubs T may be stacked onto another horizontal row of tubs along the vertical axis A during storage or transport. Typically, a cardboard C may be placed between two rows of tubs, and all the different tubs are disposed in a unique big box. When one wants to remove a tub from said big box, he needs to remove all the tubs in order to have access to all of them. Moreover, nothing prevents the axial and lateral movements of tubs with respects to other tubs. Lastly, when the cardboard is not present, the stacking of the tubs involves the cover film of an upper tub to rest on the bottom of a lower tub. The weight of this upper tub, and of any other tub stacked onto this upper tub, may cause damage to the cover film. There is therefore a need to protect the cover films during transport or storage of the medical containers, and to improve the handling of the tubs comprising said medical containers.

Besides, each tub is usually enclosed within an individual sealing bag, also called header bag, referenced B in the Figure 1. Header bags are disclosed for instance by the document EP2119463. This sealing bag helps maintain sterility of the medical containers contained in the tub. Said sealing bag needs to be opened for removing the tub and there is thus as many sealing bags as tubs to open in order to remove the medical containers. This involves several steps when removing the tubs from the big box.

In this context, an object of the present invention is to provide a spacer structure that alleviates the above-mentioned drawbacks by allowing to protect the cover films and by preventing the axial and lateral movements of the tubs during transport.A first aspect of the invention is a spacer structure configured to be arranged between a first tub and a second tub stacked onto each other along a central vertical axis A, said first tub and said second tub being configured for the transport or storage of a plurality of medical containers such as syringes, wherein the spacer structure comprises:
- a supporting frame configured to extend between an upper end of the first tub and a bottom of the second tub, the supporting frame having an outer peripheral edge;
- first maintaining means protruding from a first side of the supporting frame and configured to prevent radial displacements of the first tub relative to the spacer structure orthogonally to the vertical axis A;
- second maintaining means, opposite to the first maintaining means with respect to the supporting frame, protruding from a second side of the supporting frame and configured to prevent radial displacements of the second tub relative to the spacer structure orthogonally to the vertical axis A.

In addition, the second maintaining means have a base end which is located on the second side of the supporting frame and which is arranged offset inwardly relative to the outer peripheral edge of the supporting frame.

The spacer structure of the invention therefore allows to stack several tubs onto each other along a vertical axis A in a stable manner due to the first and second maintaining means. It is therefore possible to pack the stacked tubs into a single header bag, or big bag, i.e. a collective sealing bag configured to enclose a plurality of tubs, so as to optimize the medical containers removal process.

Moreover, having the base end of the second maintaining means offset inwardly relative to the outer peripheral edge of the supporting frame allows better fitting, and therefore better maintaining, tubs that are not necessarily box-shaped but which include a peripheral wall and an outward portion such as a flange, typically at an upper end of the tub.

In an embodiment, the supporting frame is plate-shaped. This improves the load distribution.

In an embodiment, the first maintaining means include a circumferential rib.

In an embodiment, the rib extends along a peripheral outer edge of the supporting frame.

Said rib can be configured to receive a flange of the first tub such that the edge of the flange abuts against an inner wall of the rib, thereby preventing lateral movements of the first tub relative to the spacer structure.

Preferably, the rib extends all around the supporting frame. This provides also stiffness to the supporting frame.

In an embodiment, the supporting frame and the rib form a L-shaped angle bar.

In an embodiment, the first side defines an abutment surface configured to abut against a peripheral flange of the first tub.

The supporting frame may have a central opening defined by an inner edge.

The central opening lightens the structure, thus reducing the overall load of the stacked tubs, protects the cover film of the first tub, and eases the circulation of a sterilization gas. The central opening can be sized and shaped to correspond to an opening of the first tub.

In an embodiment, the second maintaining means include protrusions configured to engage parts of a peripheral wall of the second tub. Thus, the protrusions may engage corners of the second tub. Alternatively or complementarily, the second maintaining means include protrusions configured to abut against a lateral wall of the second tub, instead of the corners.

In an embodiment, the protrusions are shaped to mate the corners of the peripheral walls of said second tub.

In an embodiment, the protrusions include an end defining an abutment surface configured to abut against the tub. The end of the protrusions may abut against an outward portion of the second tub which extends outwardly from a peripheral wall of the second tub. Said outward portion can be a flange, for example intended for sealing a cover film. Alternatively, said outward portion can be a shoulder.

In an embodiment in which the supporting frame has a central opening, the second maintaining means may be located between the outer edge and the inner edge of the supporting frame, i.e. away from both the inner and outer edges.

In an embodiment, the first and/or the second maintaining means are substantially orthogonal to the supporting frame.

The first and/or second maintaining means may include snap-fitting means for removably attaching the first tub and/or the second tub to the spacer structure. This permits to avoid inadvertent vertical movements of the tubs relative to the spacer structure.

In an embodiment, the spacer structure is made of a polymer material, a cellulose material such as molded cellulose, or a combination thereof.

In an embodiment, the spacer structure is made of a single piece.

Another aspect of the invention is a packaging including a first tub and a second tub, the second tub being stacked onto the first tub along a vertical axis A, and a spacer structure according to any of claims 1 to 12, wherein the spacer structure is arranged between a cover film of the first tub and a bottom of the second tub.

In one embodiment, this packaging has the following features:
- each of the first tub and the second tub has a peripheral wall which includes lateral walls and corners defined by two adjacent lateral walls, and at least one outward portion which extends outwardly from the peripheral wall near an upper end of said tub;
- the first maintaining means of the spacer structure fit the outer edge of the first tub at the upper end thereof;
- the second maintaining means of the spacer structure engage the peripheral wall of the second tub;

In addition, an end of the second maintaining means can define an abutment surface configured to abut against the or one of the outward portion(s) of the second tub. Alternatively or in addition, the second tub bottom wall can rest on the supporting frame of the spacer structure.

The tub may comprise a flange extending outwardly from said peripheral wall and, optionally, a shoulder, the flange and shoulder each forming one outward portion. The outer edge at the upper end of the first tub can be the outer edge of the flange.

By "engage the peripheral wall of the second tub" is meant that the second maintaining means of the spacer structure can engage the lateral walls and/or the corners of the tub.

In an embodiment, the packaging includes a single sealing bag enclosing at least the first tub, the second tub and the spacer structure.

The invention and the advantages arising therefrom will clearly emerge from the detailed description that is given below with reference to the appended drawings as follows:
- Figure 1 is a perspective view of stacked tubs,
- Figure 2 is a perspective view of an opened and empty tub,
- Figure 3 is a perspective view of an opened tub for storage and transport of a plurality of medical containers supported by a nest;
- Figure 4 is a perspective of a spacer structure according to an embodiment of the invention;
- Figure 5 is a perspective view of a packaging including the spacer structure of figure 4, according to an embodiment of the invention;
- Figure 6 is a perspective view of a spacer structure according to another embodiment of the invention;
- Figure 7 is a perspective view of a packaging including the spacer structure of figure 6, according to an embodiment of the invention.

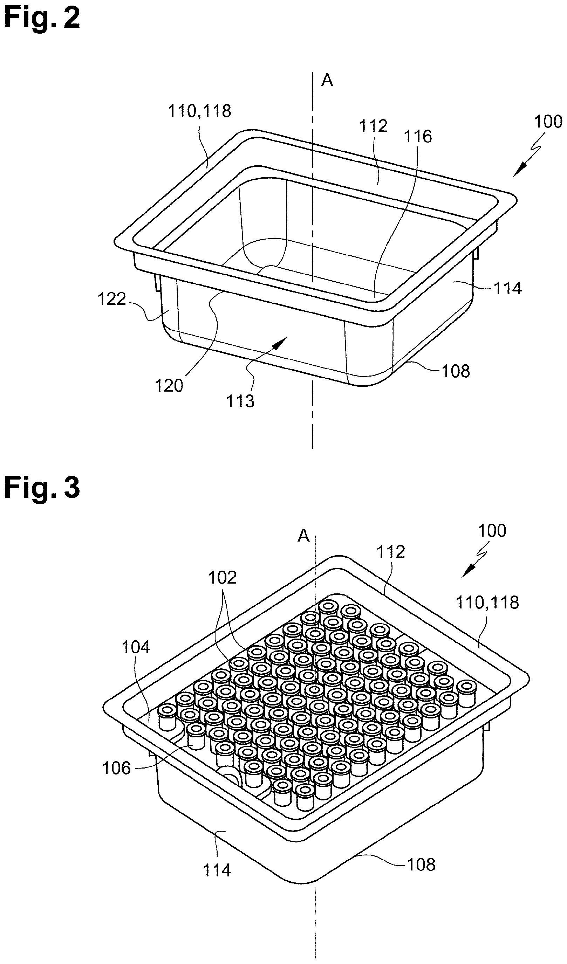

With reference to Figures 2 and 3 is shown a box-shaped housing, hereafter the tub 100, for storage and transport of a plurality of medical containers 102, such as pre-filled or prefillable syringes. The medical containers 102 are supported on a plate-shaped tray, the nest 104, that is contained inside the tub 100. The nest 104 may be inserted inside the tub 100, or removed from the tub 100, by a vertical movement along a vertical axis A. The medical containers 102 are maintained parallel to said vertical axis A by means of apertures and guiding elements 106 provided on the nest 104.

The tub 100 includes a bottom end 108 and an opposite upper end 110, said upper end 110 defining an opening 112. The bottom and upper ends 108, 110 are connected by a peripheral wall 13 which includes lateral walls 114, wherein two contiguous lateral walls 114 define a corner 122. The lateral walls 114 may taper towards the bottom end 108 and said bottom end 108 may include a closed bottom wall 116. The upper end 110 may be provided with a flange 118 and the opening 112 is usually sealed by a cover film, typically a Tyvek^{®} film (not shown). The cover film is substantially orthogonal to the vertical axis A. The cover film is permeable to sterilization gases, such as ethylene oxide (EtO) and is attached, for example glued, onto the peripheral flange 118 of the tub 100. In the removal process of the nest 104 from the tub 100, the cover film is manually or automatically peeled off. The lateral walls 114 may further include an enlarged portion near the upper end 110, which forms a shoulder 120. The flange 118 and shoulder 12 form outward portions of the tub 100, which extend outwardly from the peripheral wall 13.

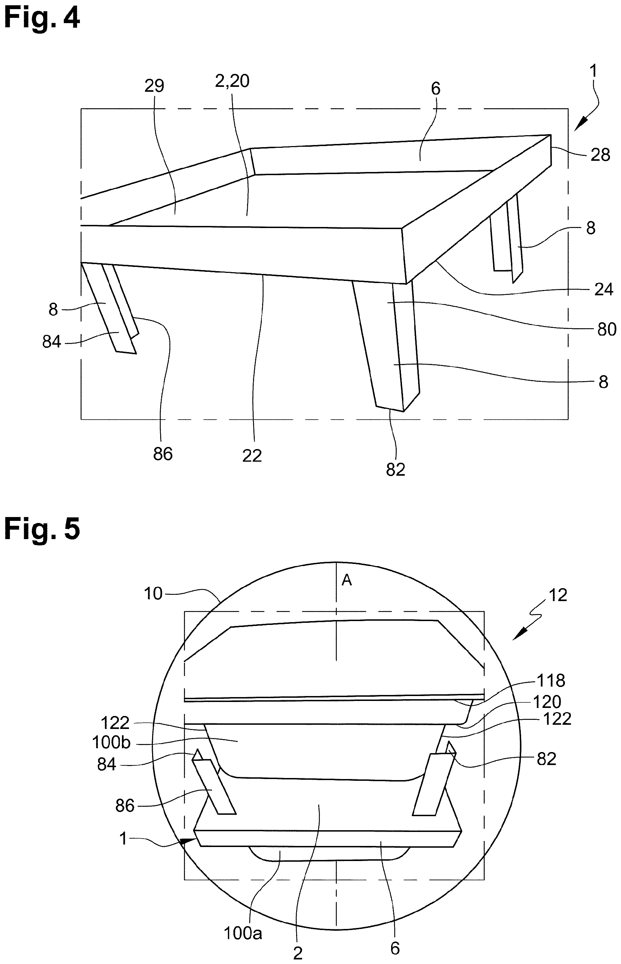

With reference to Figures 4 and 5 is shown a spacer structure 1 according to an embodiment of the invention. The spacer structure 1 is intended to separate a first and a second tubs 100a, 100b stacked onto each other so as to maintain these two tubs 100a, 100b in a stable manner with respect to the vertical axis A and to protect the cover film of one of these two tubs 100a, 100b by distributing the load.

The spacer structures 1 includes a supporting frame 2 having a first side 20 which, in use, faces the cover film or the flange 118 of the first tub 100a and an opposite second side 22 which, in use, faces the bottom 116 of the second tub 100b. The first side 20 may be configured to abut against the cover film or the flange 118 of the first tub 100a. The second side 22 may be configured to abut against the bottom 116 of the second tub 100b. The supporting frame 2 has an outer peripheral edge 24 that may define, for instance, a square or rectangular contour similar to a contour of the top of the first tub 100a. The supporting frame 2 is preferably plate-shaped in order to improve the load distribution. The first side 20 of the supporting frame defines a flat abutment surface that is intended to bear against a top face of the first tub flange 118. The load is thus transmitted to the flange 118, while the cover film may bear only a small part of the load or even no load at all.

The spacer structure 1 includes first maintaining means, for instance in the form of a peripheral rib 6, configured to prevent radial displacements of the first tub 100a relative to the spacer structure 1 and orthogonally to the vertical axis A. The first maintaining means protrude from the first side 20 of the supporting frame 2.

More specifically, the rib 6 can be located at the outer peripheral edge 24 of the supporting frame 2, and preferably extends all along this outer peripheral edge 24, thereby defining an accommodation space for receiving a top portion of the first tub 100a. The supporting frame 2 and the rib 6 may form an angle bar. The rib 6 can be substantially orthogonal to the supporting frame 2.

It is contemplated that the rib 6 and the top portion of the first tub 100a may be complementarily shaped. As visible in the Figures, the rib 6 can be orthogonal to the supporting frame 2 so as to ease the positioning or the removal of the first tub 100a relative to the spacer structure 1.

The spacer structure 1 includes second maintaining means, for instance in the form of protrusions 8, configured to prevent radial displacements of the second tub 100b relative to the spacer structure 1 and orthogonally to the vertical axis A. These second maintaining means protrude from the second side 22 of the supporting frame 2. The protrusions 8 have a base end 80 located on the second side 22 of the supporting frame 2, said base end 80 being arranged offset inwardly relative to the outer peripheral edge 24 of the supporting frame 2.

The base end 80 may be offset relative to the outer peripheral edge 24 along a direction by a distance d that is comprised between 3% and 15% of the dimension of the supporting frame 2 along said direction. For example, with an outer peripheral edge 24 having a rectangular contour, if the base end 80 is offset from a side of the outer peripheral edge 24, the "dimension of the supporting frame 2 along said direction" is the distance between the corresponding opposite sides of the rectangle, i.e. the length of the long or of the short side of the rectangle. Alternatively, if the base end 80 is offset from a corner of the outer peripheral edge 24, the "dimension of the supporting frame 2 along said direction" is the length of the diagonal of the rectangle.

Such an offset, preferably when it is adapted to the geometry and dimensions of the tub 100, makes it possible for the protrusions 8 both to cooperate with the peripheral wall 13 of the tub 100 and with the shoulder 120 or flange 118 as will be explained hereinafter.

The protrusions 8 further have an opposite free end 82. This free end 82 may be configured to abut against the shoulder 120 or the flange 118 of the tub 100b to provide a better load distribution. The protrusions 8 extend substantially orthogonal to the supporting frame 2 in order to ease the positioning or the removal of the second tub 100b relative to the spacer structure 1. More specifically, the protrusions may be slightly slanted with regard to the vertical axis A; as visible in Figure 5, they may be configured to extend parallel to the second tub 100b lateral wall 114. For instance, the angle between the protrusions 8 and the supporting frame 2 may be around 93°.

Preferably, the protrusions are long members such as legs, and not small prominences, so as to extend along at least half the height of a tub 100, or even 75% of the height of a tub 100.

As illustrated in the Figures 4 and 5, the protrusions 8 and the corners 122 may be complementarily-shaped such that the protrusions 8 are configured to mate the corners 122 of the second tub 100b. To that end, the protrusions 8 may be corner-shaped and thus comprise two orthogonal lateral walls 84, 86 preventing lateral movements of the second tub 100b in two horizontal directions. The protrusions 8 may have a curved inner surface. They are located near the corners 28 of the rectangular or square-shaped supporting frame 2. In another embodiment (not shown), the protrusions 8 may however be configured to abut against the lateral wall 114 of the second tub 100b (instead of its corners 122). In such a case, the protrusions 8 may be plate-shaped and may be located on lateral segments 29 of the rectangular or square-shaped supporting frame 2.

Although not shown in the Figures, the spacer structure 1 may have snap-fitting means for removably attaching the first tub 100a and/or the second tub 100b to the spacer structure 1.

Preferably, the spacer structure 1 is made of a polymer material or a cellulose material, such as molded cellulose. For instance, the spacer structure 1 may be of polypropylene PP, polystyrene PS, polyethylene terephthalate PET, acrylonitrile-butadiene-styrene ABS, polycarbonate PC, or combinations thereof.

Preferably, the supporting frame 2, the protrusions 8 and the peripheral rib 6 are made of a single piece.

The Figure 5 shows a packaging including a second tub 100b being positioned to be stacked onto a first tub 100a along a vertical axis A, said first and second tubs 100a, 100b being separated and maintained aligned with respect to the vertical axis A by means of the spacer structure 1. The peripheral rib 6 engages the edge of the flange 118 of the first tub 100a while the protrusions engage the corners of the second tub 100b to prevent lateral movements of this second tub 100b. The supporting frame 2 is interposed between the first and second tubs 100a, 100b, thereby distributing the load and protecting the cover film of the first tub 100a.

The bottom wall 116 of the second tub 100b may rest on the first side 20 of the supporting frame 2 and/or the flange 118 or shoulder 120 may rest on the free ends 82 of the protrusions 8.

In the packaging shown in figure 5, the second tub 100b is illustrated in an intermediate position during the stacking movement.

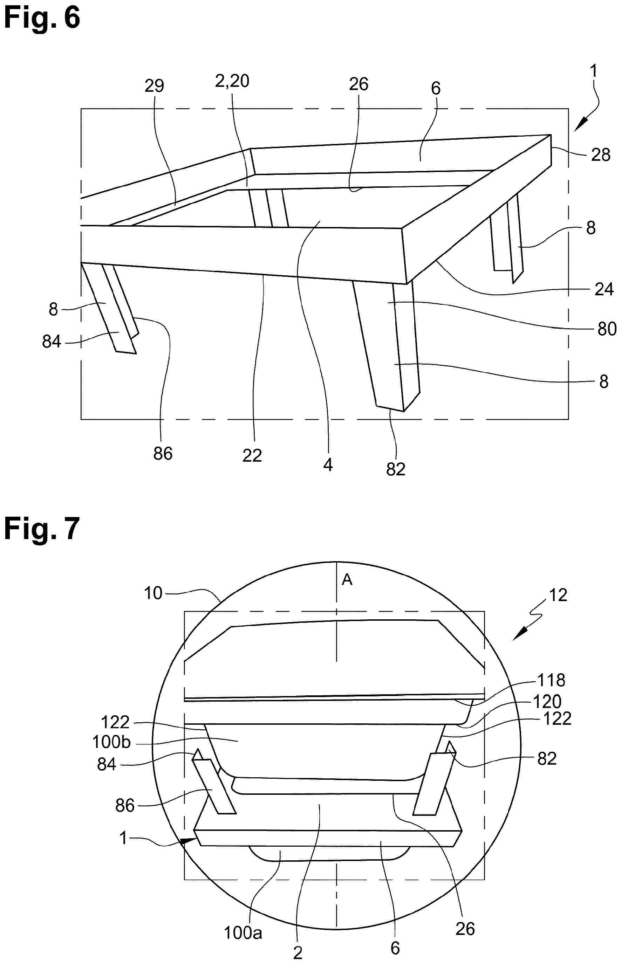

Figures 6 and 7 show another embodiment of the invention, in which the supporting frame 2 further has a central opening 4 defined by an inner edge 26. The inner edge 26 may have a square or rectangular contour

The central opening 4 improves the circulation of a sterilization gas, in particular through the cover film as said cover film is thus left at least partially uncovered by the spacer structure 1.

Besides, owing to the central opening 4, the load can be transmitted to the flange 118, but not to the cover film, via the first side 20. The cover film may only face the central opening 4. That is, the central opening 4 may be sized and shaped to correspond to the opening 112 of the first tub 100a.

The central opening 4 further allows reducing the required quantity of material used for manufacturing the spacer structure 1.

With such a configuration, the base end 80 of the protrusions 8 are located on the second side 22 of the supporting frame 2 between the outer and inner edges 24, 26.

Thanks to the spacer structure 1, the stack of the first and second tubs 100a, 100b is stabilized. The cardboard used in order to stack several tubs on a same row is not necessary anymore, and thus it is possible to enclose a line of these tubs 100a, 100b in a single sealing bag 10. It is contemplated that the packaging 8 may comprise more than two stacked tubs 100, such as for instance a stack of three tubs separated by two spacer structures 1 or a stack of four tubs 100 separated by three spacer structures 1.

## Claims

1. A spacer structure (1) configured to be arranged between a first tub (100a) and a second tub (100b) stacked onto each other along a central vertical axis A, said first tub (100a) and said second tub (100b) being configured for the transport or storage of a plurality of medical containers (102) such as syringes, wherein the spacer structure (1) comprises:
- a supporting frame (2) configured to extend between an upper end (110) of the first tub (100a) and a bottom (116) of the second tub (100b), the supporting frame (2) having an outer peripheral edge (24);
- first maintaining means (6) protruding from a first side (20) of the supporting frame (2) and configured to prevent radial displacements of the first tub (100a) relative to the spacer structure (1) orthogonally to the vertical axis A;
- second maintaining means (8), opposite to the first maintaining means (6) with respect to the supporting frame (2), protruding from a second side (22) of the supporting frame (2) and configured to prevent radial displacements of the second tub (100b) relative to the spacer structure (1) orthogonally to the vertical axis A;
**characterized in that** the second maintaining means (8) have a base end (80) which is located on the second side (22) of the supporting frame (2) and which is arranged offset inwardly relative to the outer peripheral edge (24) of the supporting frame (2).

2. The spacer structure (1) according to claim 1, wherein the supporting frame (2) is plate-shaped.

3. The spacer structure (1) according to claim 1 or 2, wherein the first maintaining means include a circumferential rib (6), which preferably extends along the peripheral outer edge (24) of the supporting frame (2).

4. The spacer structure (1) according to claim 3, wherein the supporting frame (2) and the rib (6) form a L-shaped angle bar.

5. The spacer structure (1) according to any of the preceding claims, wherein the first side (20) defines an abutment surface configured to abut against a peripheral flange (118) of the first tub (100a).

6. The spacer structure (1) according to any of the preceding claims, wherein the supporting frame (2) has a central opening (4) defined by an inner edge (26) and wherein, preferably, the central opening (4) is sized and shaped to correspond to an opening (112) of the first tub (100a).

7. The spacer structure (1) according to any of the preceding claims, wherein the second maintaining means include protrusions (8) configured to engage parts of a peripheral wall (13) of the second tub (100b), such as lateral walls (14) or corners (22) of the second tub (100b).

8. The spacer structure (1) according to claim 7, wherein the protrusions (8) are shaped to mate corners (122) of the peripheral wall (13) of said second tub (100b).

9. The spacer structure (1) according to claim 7 or 8, wherein the protrusions (8) include an end (82) defining an abutment surface configured to abut against the second tub (100b), preferably against an outward portion of the second tub (100b) which extends outwardly from a peripheral wall (13) of the second tub (100b).

10. The spacer structure (1) according to any of the preceding claims, wherein the first and/or the second maintaining means (6, 8) are substantially orthogonal to the supporting frame (2).

11. The spacer structure (1) according to any of the preceding claims, wherein the spacer structure (1) is made of a polymer material, a cellulose material such as molded cellulose, or a combination thereof.

12. The spacer structure (1) according to any of the preceding claims, wherein the spacer structure (1) is made of a single piece.

13. A packaging (12) including a first tub (100a) and a second tub (100b), the second tub (100b) being stacked onto the first tub (100a) along a vertical axis A, and a spacer structure (1) according to any of the preceding claims, wherein the spacer structure (1) is arranged between a cover film of the first tub (100a) and a bottom of the second tub (100b).

14. A packaging (12) according to claim 13, wherein:
- each of the first tub (100a) and the second tub (100b) has a peripheral wall (13) which includes lateral walls (14) and corners (22) defined by two adjacent lateral walls (14), and at least one outward portion (120, 118) which extends outwardly from the peripheral wall (13) near an upper end (110) of said tub (100a, 100b);
- the first maintaining means (6) of the spacer structure (1) fit the outer edge of the first tub (100a) at the upper end (110) thereof;
- the second maintaining means (8) of the spacer structure (1) engage the peripheral wall (13) of the second tub (100b);
and wherein, preferably, an end (82) of the second maintaining means (8) define an abutment surface configured to abut against the or one of the outward portion(s) (120, 118) of the second tub (100b).

15. A packaging (12) according to claim 13 or 14, wherein the packaging includes a single sealing bag (10) enclosing at least the first tub (100a), the second tub (100b) and the spacer structure (1).

## Patentansprüche

1. Abstandshalterstruktur (1), die dazu konfiguriert ist, zwischen einer ersten Wanne (100a) und einer zweiten Wanne (100b) angeordnet zu werden, die entlang einer zentralen vertikalen Achse A aufeinandergestapelt sind, wobei die erste Wanne (100a) und die zweite Wanne (100b) für den Transport oder die Lagerung einer Vielzahl von medizinischen Behältern (102), wie beispielsweise Spritzen, konfiguriert sind, wobei die Abstandshalterstruktur (1) Folgendes umfasst:
- Einen Stützrahmen (2), der dazu konfiguriert ist, sich zwischen einem oberen Ende (110) der ersten Wanne (100a) und einem Boden (116) der zweiten Wanne (100b) zu erstrecken, wobei der Stützrahmen (2) einen äußeren Umfangsrand (24) aufweist;
- ein erstes Haltemittel (6), das von einer ersten Seite (20) des Stützrahmens (2) hervorsteht und dazu konfiguriert ist, radiale Verschiebungen der ersten Wanne (100a) im Verhältnis zur Abstandshalterstruktur (1) orthogonal zur vertikalen Achse A zu verhindern;
- ein zweites Haltemittel (8), das dem ersten Haltemittel (6) in Bezug auf den Stützrahmen (2) gegenüberliegt, von einer zweiten Seite (22) des Stützrahmens (2) hervorsteht und dazu konfiguriert ist, radiale Verschiebungen der zweiten Wanne (100b) im Verhältnis zur Abstandshalterstruktur (1) orthogonal zur vertikalen Achse A zu verhindern;
**dadurch gekennzeichnet, dass** das zweite Haltemittel (8) ein Basisende (80) aufweist, das sich auf der zweiten Seite (22) des Stützrahmens (2) befindet und das im Verhältnis zum äußeren Umfangsrand (24) des Stützrahmens (2) nach innen versetzt angeordnet ist.

2. Abstandshalterstruktur (1) nach Anspruch 1, wobei der Stützrahmen (2) plattenförmig ist.

3. Abstandshalterstruktur (1) nach Anspruch 1 oder 2, wobei das erste Haltemittel eine umlaufende Rippe (6) enthält, die sich vorzugsweise entlang des äußeren Umfangsrands (24) des Stützrahmens (2) erstreckt.

4. Abstandshalterstruktur (1) nach Anspruch 3, wobei der Stützrahmen (2) und die Rippe (6) einen L-förmigen Winkelstab bilden.

5. Abstandshalterstruktur (1) nach einem der vorhergehenden Ansprüche, wobei die erste Seite (20) eine Anschlagfläche definiert, die dazu konfiguriert ist, an einem Umfangsflansch (118) der ersten Wanne (100a) anzuliegen.

6. Abstandshalterstruktur (1) nach einem der vorhergehenden Ansprüche, wobei der Stützrahmen (2) eine zentrale Öffnung (4) aufweist, die durch einen Innenrand (26) definiert ist, und wobei die zentrale Öffnung (4) vorzugsweise derart dimensioniert und geformt ist, dass sie einer Öffnung (112) der ersten Wanne (100a) entspricht.

7. Abstandshalterstruktur (1) nach einem der vorhergehenden Ansprüche, wobei das zweite Haltemittel Vorsprünge (8) enthält, die dazu konfiguriert sind, mit Teilen einer Umfangswand (13) der zweiten Wanne (100b), wie beispielsweise Seitenwänden (14) oder Ecken (22) der zweiten Wanne (100b), in Eingriff zu gelangen.

8. Abstandshalterstruktur (1) nach Anspruch 7, wobei die Vorsprünge (8) derart geformt sind, dass mit Ecken (122) der Umfangswand (13) der zweiten Wanne (100b) zusammenpassen.

9. Abstandshalterstruktur (1) nach Anspruch 7 oder 8, wobei die Vorsprünge (8) ein Ende (82) enthalten, das eine Anschlagfläche definiert, die dazu konfiguriert ist, an der zweiten Wanne (100b) anzuliegen, vorzugsweise an einem äußeren Abschnitt der zweiten Wanne (100b), der sich von einer Umfangswand (13) der zweiten Wanne (100b) nach außen erstreckt.

10. Abstandshalterstruktur (1) nach einem der vorhergehenden Ansprüche, wobei das erste und/oder das zweite Haltemittel (6, 8) im Wesentlichen orthogonal zum Stützrahmen (2) sind.

11. Abstandshalterstruktur (1) nach einem der vorhergehenden Ansprüche, wobei die Abstandshalterstruktur (1) aus einem Polymermaterial, einem Zellulosematerial wie geformter Zellulose oder einer Kombination davon besteht.

12. Abstandshalterstruktur (1) nach einem der vorhergehenden Ansprüche, wobei die Abstandshalterstruktur (1) einstückig hergestellt ist.

13. Verpackung (12), die eine erste Wanne (100a) und eine zweite Wanne (100b), wobei die zweite Wanne (100b) entlang einer vertikalen Achse A auf die erste Wanne (100a) gestapelt ist, und eine Abstandshalterstruktur (1) nach einem der vorhergehenden Ansprüche enthält, wobei die Abstandshalterstruktur (1) zwischen einer Abdeckfolie der ersten Wanne (100a) und einem Boden der zweiten Wanne (100b) angeordnet ist.

14. Verpackung (12) nach Anspruch 13, wobei:
- Die erste Wanne (100a) und die zweite Wanne (100b) jeweils eine Umfangswand (13) aufweisen, die Seitenwände (14) und Ecken (22), die durch zwei benachbarte Seitenwände (14) definiert sind, und mindestens einen äußeren Abschnitt (120, 118) enthält, der sich von der Umfangswand (13) in der Nähe eines oberen Endes (110) der Wanne (100a, 100b) nach außen erstreckt;
- das erste Haltemittel (6) der Abstandshalterstruktur (1) auf dem äußeren Rand der ersten Wanne (100a) an deren oberem Ende (110) sitzt;
- das zweite Haltemittel (8) der Abstandshalterstruktur (1) mit der Umfangswand (13) der zweiten Wanne (100b) in Eingriff gelangt;
und wobei vorzugsweise ein Ende (82) des zweiten Haltemittels (8) eine Anschlagfläche definiert, die dazu konfiguriert ist, an den oder einen der äußeren Abschnitte (120, 118) der zweiten Wanne (100b) anzuliegen.

15. Verpackung (12) nach Anspruch 13 oder 14, wobei die Verpackung einen einzelnen Verschlussbeutel (10) enthält, der mindestens die erste Wanne (100a), die zweite Wanne (100b) und die Abstandshalterstruktur (1) umschließt.

## Revendications

1. Structure d'espacement (1) configurée pour être agencée entre une première cuve (100a) et une deuxième cuve (100b) empilées l'une sur l'autre le long d'un axe vertical central A, ladite première cuve (100a) et ladite deuxième cuve (100b) étant configurées pour le transport ou le stockage d'une pluralité de récipients médicaux (102) tels que des seringues, dans laquelle la structure d'espacement (1) comprend :
- un cadre de support (2) configuré pour s'étendre entre une extrémité supérieure (110) de la première cuve (100a) et un fond (116) de la deuxième cuve (100b), le cadre de support (2) ayant un bord périphérique externe (24) ;
- des premiers moyens de maintien (6) faisant saillie d'un premier côté (20) du cadre de support (2) et configurés pour empêcher des déplacements radiaux de la première cuve (100a) par rapport à la structure d'espacement (1) orthogonalement à l'axe vertical A ;
- des deuxièmes moyens de maintien (8), opposés aux premiers moyens de maintien (6) par rapport au cadre de support (2), faisant saillie d'un deuxième côté (22) du cadre de support (2) et configurés pour empêcher des déplacements radiaux de la deuxième cuve (100b) par rapport à la structure d'espacement (1) orthogonalement à l'axe vertical A ;
**caractérisée en ce que** les deuxièmes moyens de maintien (8) ont une extrémité de base (80) qui est située sur le deuxième côté (22) du cadre de support (2) et qui est agencée en étant décalée vers l'intérieur par rapport au bord périphérique externe (24) du cadre de support (2).

2. Structure d'espacement (1) selon la revendication 1, dans laquelle le cadre de support (2) est en forme de plaque.

3. Structure d'espacement (1) selon la revendication 1 ou 2, dans laquelle les premiers moyens de maintien comprennent une nervure circonférentielle (6), qui s'étend de préférence le long du bord périphérique externe (24) du cadre de support (2).

4. Structure d'espacement (1) selon la revendication 3, dans laquelle le cadre de support (2) et la nervure (6) forment une cornière en forme de L.

5. Structure d'espacement (1) selon l'une des revendications précédentes, dans laquelle le premier côté (20) définit une surface de butée configurée pour venir en butée contre un rebord périphérique (118) de la première cuve (100a).

6. Structure d'espacement (1) selon l'une des revendications précédentes, dans laquelle le cadre de support (2) a une ouverture centrale (4) définie par un bord interne (26) et dans laquelle, de préférence, l'ouverture centrale (4) est dimensionnée et mise en forme pour correspondre à une ouverture (112) de la première cuve (100a).

7. Structure d'espacement (1) selon l'une des revendications précédentes, dans laquelle les deuxièmes moyens de maintien comprennent des saillies (8) configurées pour s'engager avec des parties d'une paroi périphérique (13) de la deuxième cuve (100b), telles que des parois latérales (14) ou des coins (22) de la deuxième cuve (100b).

8. Structure d'espacement (1) selon la revendication 7, dans laquelle les saillies (8) sont mises en forme pour s'adapter aux coins (122) de la paroi périphérique (13) de ladite deuxième cuve (100b).

9. Structure d'espacement (1) selon la revendication 7 ou 8, dans laquelle les saillies (8) comprennent une extrémité (82) définissant une surface de butée configurée pour venir en butée contre la deuxième cuve (100b), de préférence contre une partie extérieure de la deuxième cuve (100b) qui s'étend vers l'extérieur à partir d'une paroi périphérique (13) de la deuxième cuve (100b).

10. Structure d'espacement (1) selon l'une des revendications précédentes, dans laquelle les premiers et/ou deuxièmes moyens de maintien (6, 8) sont sensiblement orthogonaux au cadre de support (2).

11. Structure d'espacement (1) selon l'une des revendications précédentes, dans laquelle la structure d'espacement (1) est constituée d'un matériau polymère, d'un matériau cellulosique tel que la cellulose moulée, ou d'une combinaison de ceux-ci.

12. Structure d'espacement (1) selon l'une des revendications précédentes, dans laquelle la structure d'espacement (1) est réalisée en une seule pièce.

13. Emballage (12) comprenant une première cuve (100a) et une deuxième cuve (100b), la deuxième cuve (100b) étant empilée sur la première cuve (100a) le long d'un axe vertical A, et une structure d'espacement (1) selon l'une des revendications précédentes, dans lequel la structure d'espacement (1) est agencée entre un film de couverture de la première cuve (100a) et un fond de la deuxième cuve (100b).

14. Emballage (12) selon la revendication 13, dans lequel :
- chacune de la première cuve (100a) et de la deuxième cuve (100b) a une paroi périphérique (13) qui comprend des parois latérales (14) et des coins (22) définis par deux parois latérales adjacentes (14), et
au moins une partie extérieure (120, 118) qui s'étend vers l'extérieur depuis la paroi périphérique (13) près d'une extrémité supérieure (110) de ladite cuve (100a, 100b) ;
- les premiers moyens de maintien (6) de la structure d'espacement (1) s'ajustent au bord externe de la première cuve (100a) au niveau de son extrémité supérieure (110) ;
- les deuxièmes moyens de maintien (8) de la structure d'espacement (1) s'engagent avec la paroi périphérique (13) de la deuxième cuve (100b) ;
et dans lequel, de préférence, une extrémité (82) du deuxième moyen de maintien (8) définit une surface de butée configurée pour venir en butée contre la ou l'une des partie(s) extérieure(s) (120, 118) de la deuxième cuve (100b).

15. Emballage (12) selon la revendication 13 ou 14, dans lequel l'emballage comprend un seul sac hermétique (10) renfermant au moins la première cuve (100a), la deuxième cuve (100b) et la structure d'espacement (1).
